# EUROPÄISCHE PATENTSCHRIFT

(11) **EP 1 464 289 B1**
(45) Veröffentlichungstag und Bekanntmachung des Hinweises auf die Patenterteilung: **27.02.2008**
(21) Anmeldenummer: 04007805.7
(22) Anmeldetag: 31.03.2004
(51) Int. Cl.: A61B 17/28

(54) **Chirurgisches Instrument, bei welchem Bewegungen des Instrumentskopfs und des Effektors entkoppelt sind**
Surgical instrument, in which movements of the head and of the effector are decoupled
Instrument chirurgical, dans lequel les mouvements de la tête et de l'outil sont découplés

(30) Priorität: 01.04.2003 DE 10314827
(43) Veröffentlichungstag der Anmeldung: 06.10.2004
(73) Patentinhaber: Tuebingen Scientific Medical GmbH, 72074 Tuebingen (DE)
(72) Erfinder: Braun, Markus, 70563 Stuttgart-Vaihingen (DE)
(74) Vertreter: TBK-Patent

(56) Entgegenhaltungen:
- DE-A- 10 036 108
- US-A- 5 540 375
- US-A- 5 820 009
- US-A- 6 010 523

## Beschreibung

Die vorliegende Erfindung betrifft ein chirugisches Instrument für die minimal invasive Chirurgie gemäß dem Oberbegriff des Patentanspruchs 1.

Aus der DE 100 36 108 ist ein chirugisches Instrument dieser Gattung bekannt. Dieses besteht im wesentlichen aus einem Rohrschaft, an dessen einem proximalen Ende ein Instrumentengriff angeordnet ist, mittels dem ein an dem gegenüberliegenden distalen Ende des Rohrschafts angeordneter Instrumentenkopf über Getriebezüge betätigbar ist. Der Instrumentenkopf läßt sich bezüglich des Rohrschafts verschwenken bzw. neigen und trägt zudem einen drehbar im Instrumentenkopf gelageren Effektor in Form einer Art Zange oder Greifer, deren eines Zangenstück schwenkbar am Effektor gelagert und mittels des Instrumentengriffs ebenfalls betätigbar ist.

Konkreter ausgedrückt ermöglichen die Getriebezüge, dass zumindest eine erste Bewegung des Instrumentengriffs, beispielsweise auslösbar durch die Handrotation einer Bedienerperson, in eine Rotation des Effektors unter einem bestimmten Übersetzungsverhältnis zu dieser Betätigungsbewegung transformiert wird. Hierdurch ist es möglich, den Effektor trotz der relativ eingeschränkten Bewegungsmöglichkeit einer menschlichen Hand um beispielsweise bis zu 300° zu drehen und damit komplizierte Bewegungsabläufe ohne Umgreifen am Handgriff zu realisieren. Des weiteren wird eine zweite Bewegung des Instrumentengriffs, beispielsweise dessen Abwinkeln bezüglich des Rohrschafts, in eine Neigungsbewegung des Instrumentenkopfs umgesetzt.

Die Getriebezüge innerhalb des Instrumentengriffs und des Rohrschafts sind derart ausgelegt, dass eine weitestgehend entkoppelte Betätigung jeder einzelnen Bewegung des Instrumentenkopfs sowie des Effektors ermöglicht wird. Allderdings sind derartige Getriebe zwangsläufig äußerst kompliziert und benötigen demzufolge auch ausreichend Bauraum. Darüber hinaus ist eine vollständige Entkopplung der einzelnen Bewegungen nicht gänzlich gewährleistet.

Das Dokument US-A-5 820 009 beschreibt ein chirurgisches Instrument mit einem Griffstück, einem Rohrschaft, einem einen Effektor lagernden Instrumentenkopf, der neigbar angelenkt ist. Der Effektor weist ein schwenkbares Eingriffselement auf, welches über einen Effektor-Betätigungsgetriebezug mittels des Griffstücks betätigbar ist. Zudem weist der Effektor-Betätigungsgetriebezug einen im Rohrschaft verschiebbar angeordneten Schubstab auf, der im Anlenkbereich zwischen Instrumentenkopf und Rohrschaft mit einem im Instrumentenkopf und Effektor verschiebbar gelagerten Schubbolzen in Anlage ist, der mit dem Eingriffselement wirkverbunden ist. Der Effektor dieses Instruments ist allerdings nicht drehbar gelagert und der Schubstab sowie der Schubbolzen sind nicht in einer Neigungsposition des Instrumentenkopfs zum Rohrschaft von 0° koaxial zueinander ausgerichtet.

Angesichts dieses Stands der Technik ist es eine Aufgabe der vorliegenden Erfindung, ein chirugisches Instrument dieser Gattung zu schaffen, bei welchem Bewegungen eines Instrumentenkopfs sowie eines Effektors voneinander entkoppelt über einen Instrumentengriff ausführbar sind.

Diese Aufgabe wird durch ein chirugisches Instrument mit den Merkmalen gemäß dem Patentanspruch 1 gelöst.

Der Kern der Erfindung besteht demzufolge darin, dass der Effektor-Betätigungsgetriebezug einen im Rohrschaft verschiebbar angeordneten Schubstab aufweist, der im Anlenkbereich des Instrumentenkopfs am Rohrschaft mit einem im Instrumentenkopf oder im Effektor selbst verschiebbar gelagerten Schubbolzen in Anlage ist, der mit dem Eingriffselement wirkverbunden ist. Hierdurch wird eine Schubbewegung unabhängig von der Neigungsposition des Instrumentenkopfs bezüglich des Rohrschafts auf den Schubbolzen übertragen, ohne dass komplizierte Umlenkgetriebe erforderlich wären.

Eine besonders vorteilhafte Ausgestaltung der Erfindung sieht vor, dass der Schubstab eine distale Stirnfläche hat, welche den Eingiffsabschnitt mit dem Schubbolzen bildet und welche unter einem vorbestimmten Winkel von vorzugsweise 45° in die Abwinkelungsrichtung des Instrumentenkopfs abgeschrägt ist. Durch diese Maßnahme wird eine Art Kraftumlenkmittel geschaffen, wodurch eine Vorschubbewegung des Schubstabs unabhängig von der Abwinkelposition des Instrumentenkopfs optimal auf den Schubbolzen übertragen werden kann.

Weitere vorteilhafte Ausgestaltungen der Erfindung sind Gegenstand der Unteransprüche.

Die Erfindung wird nachstehend anhand eines bevorzugten Ausführungsbeispiels unter Bezugnahme der begleitenden Zeichnungen näher erläutert.
Fig. 1 zeigt eine Perspektivenansicht eines chirurgischen Instruments gemäß einem bevorzugten Ausführungsbeispiels der Erfindung,
Fig. 2 zeigt einen ersten Getriebezug für ein Verschwenken eines Instrumentenkopfs mittels eines Instrumentengriffs,
Fig. 3 zeigt eine Teilschnittansicht des ersten Getriebezugs im Schwenkbereich des Instrumentenkopfs,
Fig. 4 zeigt einen zweiten Getriebezug für ein Rotieren des Instrumentenkopfs mittels des Instrumentengriffs,
Fig. 5 zeigt eine Teilschnittansicht des zweiten Getriebezugs im Schwenkbereich des Instrumentenkopfs und
Fig. 6a-6c zeigen Schnittdarstellungen eines dritten Getriebezugs im Schwenkbereich des Instrumentenkopfs zur Betätigung einer am Instrumentenkopf gelagerten Zange.

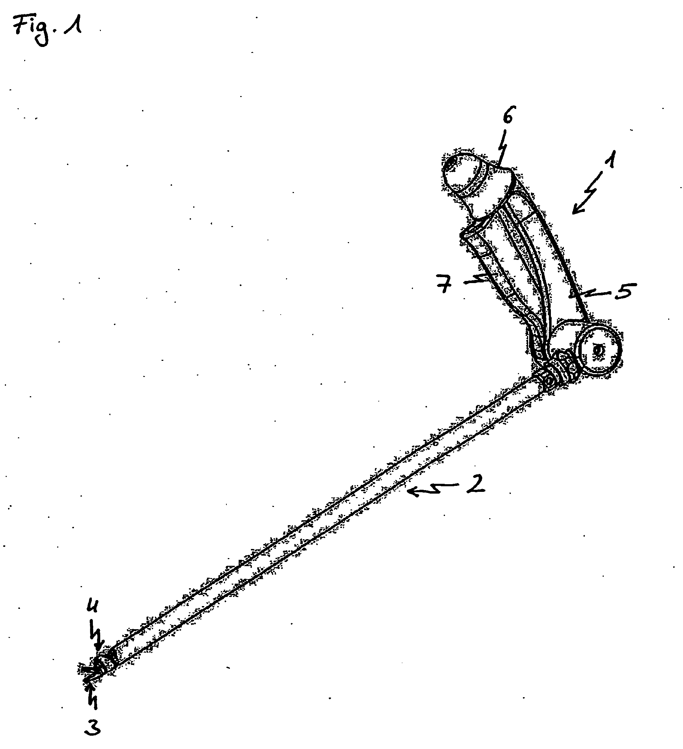

In der Fig. 1 ist ein vollständiges chirurgisches Instrument gemäß einem bevorzugten Ausführungsbeispiel der Erfindung in einer Perspektivenansicht dargestellt. Das erfindungsgemäße chirurgische Instrument hat demzufolge einen multifunktionalen Instrumentengriff 1, der an einem proximalen Ende oder Endabschnitt eines vorzugsweise aus nichtrostendem Stahl, einer Stahllegierung oder einem Kunststoffmaterial gefertigten Rohrschafts 2 angeordnet ist, sowie einem mit einem Effektor 3 bestückten oder bestückbaren Instrumentenkopf 4, der an dem anderen, distalen Ende des Rohrschafts 2 vorgesehen ist.

Generell ist der Instrumentenkopf 4 an dem betreffenden Rohrschaftende so gelagert, dass er bezüglich des Rohrschafts 2 verschwenkt bzw. abgewinkelt werden kann, wohingegen der Effektor 3 in jeder Abwinkelposition des Instrumentenkopfs 4 um dessen Längsachse gedreht bzw. rotiert werden kann, wobei die beiden vorstehend genannten Bewegungen mittels des Instrumentengriffs 1 ausführbar sind. Hierzu sind am Instrumentengriff 1 eine Anzahl von Handhaben oder Betätigungsmechanismen vorgesehen, die über entsprechende Getriebezüge innerhalb des Instrumentengriffs 1 sowie des Rohrschafts 2 mit dem Instrumentenkopf 4 bzw. dem Effektor 3 wirkverbunden sind, um die einzelnen Bewegungen des Instrumentenkopfs 4 und des Effektors 3 unabhängig voneinander, d.h. entkoppelt ausführen zu können.

Konkret besteht der Instrumentengriff 1 aus einem ergomisch geformten Griffstück 5, das schwenk- bzw. neigbar am Rohrschaft 2 montiert ist und an dem eine erste Handhabe 6, vorliegend vorzugsweise in Form eines Drehknopfs sowie eine zweite Handhabe 7, vorliegend vorzugsweise in Form eines Griffhebels gelagert sind. Somit besitzt der Instrumentengriff 1 gemäß dem bevorzugten Ausführungsbeispiel der vorliegenden Erfindung insgesamt drei Betätigungsmechanismen für drei unabhängige Bewegungen des bzw. am Instrumentenkopf 4. An dieser Stelle sei ausdrücklich darauf hingewiesen, dass der Instrumentengriff 1 auch weniger Betätigungsmöglichkeiten aufweisen kann, etwa jeweils nur einen Betätigungsmechanismus für ein Verschwenken des Instrumentenkopfs 4 und Drehen des Effektors 3.

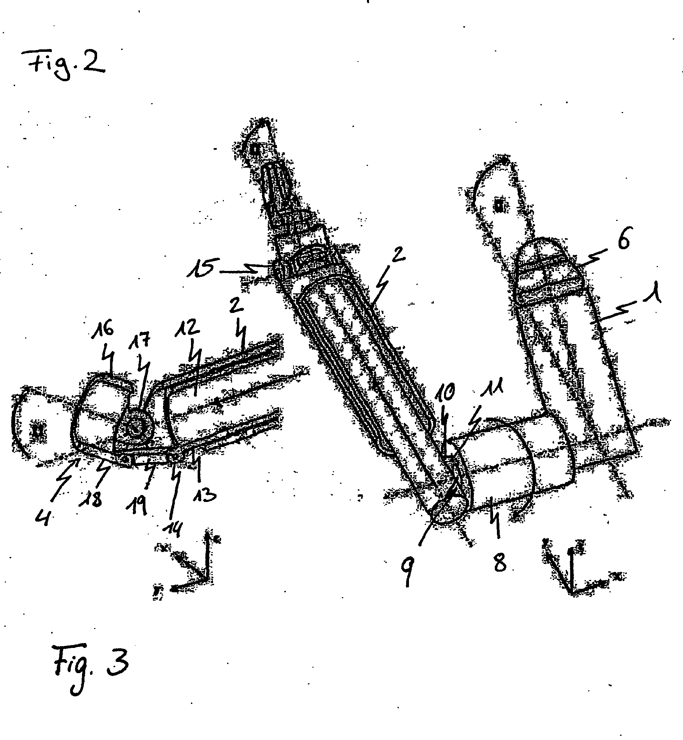

Der äußere Aufbau des Instrumentengriffs 1 insbesondere mit Bezug auf den Betätigungsmechanismus für ein Verschwenken bzw. Abwinkeln des Instrumentenkopfs 4 sowie den zugehörigen Abwinklungs-Getriebezug ist in den Fig. 2 und 3 dargestellt.

Das in Fig. 2 idealisiert dargestellte Griffstück 1 ist über ein fest mit dem Griffstück 1 verbundenes Kulissenelement 8 in Form einer Drehwelle oder Drehscheibe mit dem Rohrschaft 2 schwenkbar verbunden. Hierbei ist die Drehwelle 8 vorzugsweise senkrecht zum Rohrschaft 2 sowie zum Griffstück 1 ausgerichtet und beabstandet das Griffstück 1 vom Rohrschaft 2 derart, dass dieses im wesentlichen parallel zum Rohrschaft 2 an diesem vorbei verschwenkt werden kann.
Die Drehwelle 8, welche einen zentralen Durchgangskanal 9 zur Aufnahme nachfolgend noch beschriebener Getriebeteile ausbildet, ist an ihrer einen, dem Rohrschaft 2 zugewandten Stirnseite mit einer Kulissenführung 10 in Form einer nockenförmigen Nut ausgebildet, in die ein Mitnehmerstift 11 eingreift, welcher an einem im Rohrschaft 2 gelagerten, axial verschiebbaren Schubrohr 12 befestigt ist. Die Nut 10 ist dabei derart ausgebildet, dass bei einer Drehbewegung der Drehwelle 8 durch entsprechendes Schwenken des Griffstücks 1 der Mitnehmerstift 11 in der Nut 10 entlang gleitet und dabei eine Zwangs-Ausgleichsbewegung in Längsrichtung des Rohrschafts 2 ausführt, die auf das Schubrohr 12 übertragen wird und je nach Drehrichtung der Drehwelle 8 zu einer Hin- und Herbewegung des Schubrohrs 12 innerhalb des Rohrschafts 2 führt.

Der dem Kulissenelement 8 abgewandte, distale Endabschnitt des Schubrohrs 12 ist mit einer längs sich ersteckenden Lasche 13 ausgebildet, die über das distale Ende des Schubrohrs 12 vorsteht und an ihrem freien Endabschnitt ein Scharnier bzw. Scharnierösen 14 bildet. Des weiteren ist die Stirnseite des Rohrschafts 2 an seinem distalen Endabschnitt in einem Winkel von vorzugsweise 45° abgeschrägt und mit seitlichen Gelenkösen 15 ausgebildet, an denen der Instrumentenkopf 4 schwenkbar über Gelenkzapfen oder Stifte angelenkt ist. Dieser besteht ebenfalls aus einem Rohrstück 16, dessen eine Stirnseite, an der Anlenkösen 17 für ein Verbinden mit dem Rohrschaft 2 bzw. dessen Gelenkösen 15 ausgebildet sind, ebenfalls in einem Winkel von vorzugsweise 45° abgeschrägt ist und zwar derart, dass sich nach einem Anlenken des Instrumentenkopfs 4 am Rohrschaft 2 die beiden vorstehend genannten Schrägen ergänzen und ein Abwinkeln des Rohrstücks 16 bezüglich des Rohrschafts 2 um ca. 90° vorzugsweise 70° ermöglichen.

Des weiteren ist an der abgeschrägten Stirnseite des Rohrstücks 16 ein Scharnier bzw. Scharnierösen 18 ausgebildet. An den schubrohrseitigen sowie den rohrstückseitigen Scharnierösen 14; 18 ist ein Kipphebel 19 jeweils anscharniert, welcher folglich zur Schwenkachse des Instrumentenkopfs 4 radial nach außen versetzt ist und eine axiale Translationsbewegung des Schubrohrs 12 auf das Rohrstück 16 überträgt, wodurch dieses um dessen Schwenkachse geschwenkt wird.

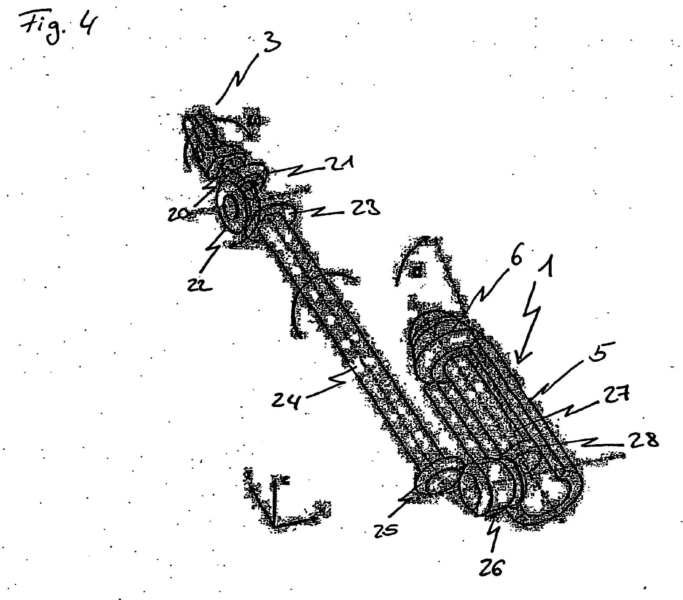

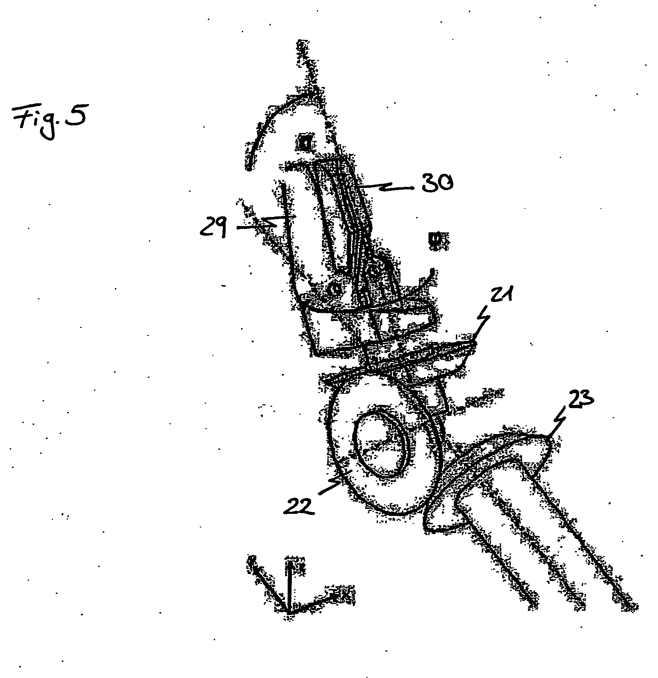

Im nachfolgenden wird anhand der Fig. 4 und 5 der Betätigungsmechanismus für eine Rotation des im Instrumentenkopf 4 gelagerten Effektors 3 sowie der zugehörige Rotations-Getriebezug beschrieben.

Wie noch aus der Fig. 2 zu entnehmen ist, stellt das vorstehend genannte Rohrstück 16 des Instrumentenkopfs 4 gleichzeitig ein Gehäuse bzw. eine Aufnahme für den Effektor 3 dar. Unabhängig davon, um welche Art Effektor es sich dabei handelt, also unabhängig davon, ob als Effektor beispielsweise ein Nadelhalter, ein Greifer, eine Zange oder Schere zum Einsatz kommt, besitzt dieser eine vorzugsweise hohle Rotationsachse 20, die in das Rohrstück 16 des Instrumentenkopfs 4 drehbar eingesetzt und gegen eine Axialbewegung gesichert ist. Die Länge dieser Rotationsachse 20 ist dabei so gewählt, dass diese in etwa im Bereich der Schwenkachse des Instrumentenkopfs 4 endet und an ihrem freien, zu dieser Schwenkachse ragenden Ende mit einem Abtriebs-Stirnrad 21 versehen ist, das drehfest an der Rotationsachse 20 des Effektors 3 befestigt ist. Insbesondere in der Fig. 2 ist die Schwenkachse des Instrumentenkopfs 4 durch eine Strich-Linie durch die Ösen 15 dargestellt.

Wie aus der Fig. 5 ferner zu entnehmen ist, befindet sich auf der Schwenkachse des Instrumentenkopfs 4 ein Drehmoment-Übertragungsstirnrad 22, welches an einem der beiden nicht weiter dargestellten Schwenkzapfen des Instrumentenkopfs 4, welche die idealisiert dargestelle Schwenkachse bilden, drehbar gelagert und mit dem Abtriebs-Stirnrad 21 in Kämmeingriff ist. Das Drehmoment-Übertragungsstirnrad 22 ist wiederum mit einem Antriebs-Stirnrad 23 in Kämmeingriff, welches auf einer innerhalb des Schubrohrs 12 (in den Fig. 4 und 5 nicht dargestellt) drehbar geführten Antriebswelle 24 drehfest montiert ist, wie dies insbesondere in der Fig. 4 gezeigt ist. An einem dem Antriebs-Stirnrad 23 gegenüberliegenden Ende der Antriebswelle 24 ist gemäß der Fig. 4 ein weiteres Drehmoment-Einleitungsstirnrad 25 drehfest angeordnet, welches mit einer Zahnradwelle 26 in Kämmeingriff ist, die in dem innerhalb des Kulissenelements 8 ausgebildeten zentralen Durchgangskanal 9 gelagert ist.

Das Kulissenelement 8 ist in der Fig. 4 nicht weiter dargestellt.

Die Zahnradwelle 26 ist schließlich mit einer Betätigungswelle 27, bzw. einem daran befestigten Stirnrad 28 innerhalb des Griffstücks 1 in Kämmeingriff, welche mit der einen Handhabe, vorliegend dem Drehknopf 6, fest verbunden ist.

Bei einer Betätigung des Drehknopfs 6 wird dessen Drehbewegung über die Betätigungswelle 27 innerhalb des Griffstücks 1, die Zahnradwelle 26, die daran sich anschließende Antriebswelle 24 innerhalb des Schubrohrs 12 sowie das Übertragungsstirnrad 22 auf den Effektor 3 übertragen und dieser gedreht. Der Drehknopf 6 wird dabei in vorteilhafter Weise mit den Fingern insbesondere mit Daumen und Zeigefinder der Bedienerhand betätigt, während das Griffstück 1 in Hand gehalten wird. Es ist somit möglich, jede beliebige Rotation am Effektor 3 zu erzeugen, ohne dass der Bediener am Griffstück 1 selbst umgreifen muss. An dieser Stelle sei ferner noch darauf hingewiesen, dass die Antriebswelle 24 sowie das Schubrohr 12 in Axialrichtung relativ beweglich zueinander gelagert sind. D.h. eine durch ein Verschwenken des Handgriffs 1 ausgelöste Drehung des Kulissenelements 8 bewirkt zwar eine Translationsbewegung des Schubrohrs 12. Gleichzeitig wird jedoch die Antriebswelle 24 in Position, d.h. in Kämmeingriff mit der Zahnradwelle 26 gehalten, wodurch das Schubrohr 12 eine axiale Relativbewegung zum Rohrschaft 2 wie auch zur Antriebswelle 24 ausführt.

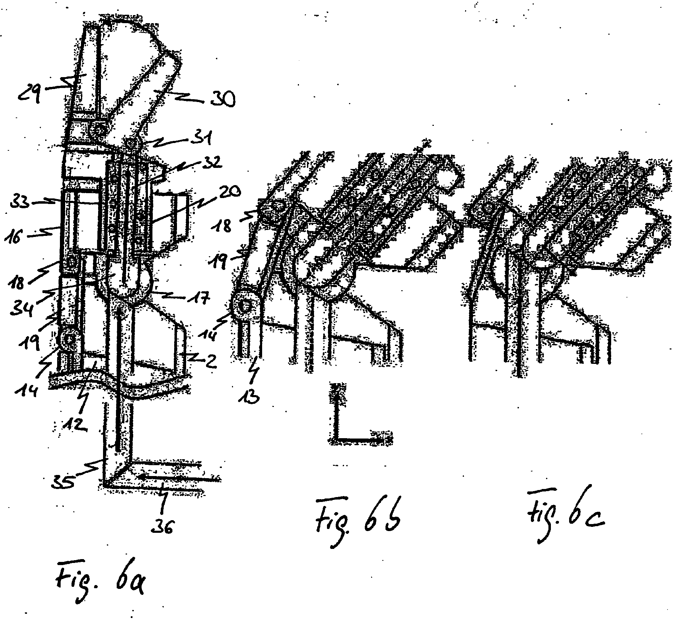

Schließlich wird nachfolgend der Betätigungsmechanismus für den Effektor 3, d.h. dessen Funktionen selbst sowie den zugehörigen Effektor-Getriebezug anhand der Fig. 5 und 6a-6c beschrieben.

Gemäß der Fig. 5 ist der Effektor 3 in dem vorliegenden Ausführungsbeispiel der Erfindung als eine Zange mit einer feststehenden sowie einer bewegbaren, d.h. schwenkbaren Zangenbacke 29; 30 ausgebildet. Die feststehende Zangenbacke 29 bildet zusammen mit der Rotationswelle 20 des Effektors 3 eine Einheit und ist vorzugsweise einstückig mit der Rotationswelle 20 ausgebildet, wohingegen die bewegbare Zangenbacke 30 an einem Ende schwenkbar an der feststehenden Zangenbacke 29 angelenkt ist.

Die bewegbare Zangenbacke 30 bildet eine Anlenkstelle 31 für einen Schubbolzen 32, der innerhalb der Rotationswelle 20 relativ verschiebbar gelagert ist, sodass durch dessen Axialverschiebung eine Schwenkbewegung der bewegbaren Zangenbacke 30 mit möglichst großer Übersetzung bewirkt wird. Der Schubbolzen 32 ist, wie dies insbesondere in den Fig. 6a - 6c dargestellt wird, mittels einer Feder 33 axial in Öffnungsrichtung der Zange vorgespannt, die innerhalb der Rotationsachse 20 den Schubbolzen 32 umgibt. Hierfür bildet der Schubbolzen 32 einen Wellenabsatz, an dem sich die Vorspannfeder 33 an ihrem einen Ende abstützt. Das andere Ende der Vorspannfeder 33 ist gegen die feststehende Zangenbacke 29 abgestützt. Ein aus der Rotationsachse 20 in Richtung zur Schwenkachse des Instrumentenkopfs 4 herausragendes Endstück 34 des Schubbolzens 32 ist kugelkopfförmig ausgebildet, wobei der Radius des Kugelkopfs 34 vorliegend vorzugsweise ca. 2.5 mm beträgt.

Die vorstehend genannte Antriebswelle 24 für das rotorische Antreiben des im Instrumentenkopf 3 gelagerten Effektors 3 ist mit einer im wesentlichen durchgehenden Axialbohrung (nicht weiter gezeigt) versehen. In dieser Axialbohrung ist ein Schubstab 35 axialverschieblich, sowie relativ zur Antriebswelle 24 drehbar geführt, dessen dem Schubbolzen 32 zugewandte Stirnseite entsprechend den Abschrägungen der rohrschaftseitigen wie auch schubrohrseiten distalen Stirnseiten d.h. vorzugsweise 45° in die selbe Richtung abgeschrägt ist. Der Schubbolzen 32 ist mittels der Feder 33 gegen diese abgeschrägte Stirnfläche des Schubstabs 35 vorgespannt und kommt mit dieser in Anlage. Dabei ist die Anlagefläche zwischen dem Schubstab 35 und dem Schubbolzen 32 aufgrund der vorstehend beschriebenden Kugelkopfform des Bolzens 32 im wesentlichen punktförmig und zwar unabhängig von dem Abwinkelungsgrad des Instrumentenkopfs 4 sowie unabhängig von der Rotationsposition des Effektors 3.

Wie aus der Fig. 6a zu erkennen ist, sind der Schubbolzen 32 wie auch der Schubstab 35 für den Fall, dass die Abwinkelung des Instrumentenkopfs 4 bezüglich des Rohrschafts 2 im wesentlichen 0° beträgt, axial zueinander ausgerichtet. Darüber hinaus ist der Schubbolzen 32 in dieser Stellung des Instrumentenkopfs 4 so positioniert, dass der Mittelpunkt des Kugelkopfs 34 des Schubbolzens 32 in etwa in der Schwenkachse des Instrumentenkopfs 4 liegt.

Der Schubstab 35 ist an seinem proximalen Ende über ein nicht im Einzelnen gezeigtes Getriebe 36 mit dem Betätigungshebel 7 verbunden, der, wie Eingangs dieser Beschreibung bereits kurz ausgeführt wurde, schwenkbar am Griffstück 1 gelagert ist.

Die Funktionsweise des erfindungsgemäßen chirurgischen Instruments wird nachfolgend im Einzelnen beschrieben.

Eine Rotation des im Instrumentenkopf 4 gelagerten Effektors 3 erfolgt durch eine Betätigung des an einem Ende des Griffstücks 1 gelagerten Drehkopfs 6, wobei der Drehknopf 6 wie vorstehend bereits ausgeführt wurde, soweit um seine Drehachse gedreht werden kann, dass eine ca. 360° Rotation am Effektor 3 realisiert wird, ohne dass am Griffstück 1 notwendiger Weise umgegriffen werden muss. Diese Drehbewegung wird über die Betätigungswelle 27 auf die Zahnradwelle 26 übertragen, welche wiederum ihre Drehbewegung auf die innerhalb des Schubrohrs 12 verlaufende Antriebswelle 24 weiter gibt. Die Drehbewegung der Antriebswelle 24 bewirkt eine Drehbewegung des Übertragungsstirnrads 22, welches die Schwenkachse des Instrumentenkopfs 4 quasi überbrückt und damit eine Rotationsbewegung des Effektors 3 innerhalb des Rohrstücks 16 des Instrumentenkopfs 4 um die Rohrstückachse auslöst.

Um eine Abwinkelung, d.h. eine Schwenkbewegung des Instrumentenkopfs 4 und damit des Effektors 3 zu bewirken, muß gemäß dem vorliegenden Ausführungsbeispiel das gesamte Griffstück 1 um die Längsachse des Kulissenelements 8 geschwenkt werden. In anderen Worten ausgedrückt, bewirkt eine Schwenkbewegung des Griffstücks 1 bezüglich des Rohrschafts 2 eine Drehbewegung des drehfest mit dem Griffstück 1 verbundenen Kulissenelements 8. Gleichzeitig jedoch wird aufgrund der Tatsache, dass durch den Kämmeingriff zwischen der Betätigungsachse 27 und der Zahnradwelle 26 eine Art Selbsthemmung durch Reibung (Wirkungsgrad des Getriebes) entsteht, welche ggf. durch leichtes Halten des Betätigungsknopfs 6 sowie durch die Haftreibung zwischen Betätigungsknopf 6 und Griffstück 1 noch unterstützt wird, die Zahnradwelle 26 mit dem Kulissenelement 8 mit gedreht.

Die Drehung des Kulissenelements 8 wird über die Kulisse bzw. Nut 10 an der Stirnseite des Elements 8 sowie den Mitnehmerzapfen 11 in eine Axialbewegung des Schubrohrs 12 übertragen, die über den anscharnierten Kipphebel 19 zu einer Schwenkbewegung des Instrumentenkopfs 4 um dessen Schwenkachse transformiert wird. Diese Schwenkbewegung führt jedoch zwangsläufig auch das Abtriebsstirnrad 21 aus, welches an der Rotationsachse des Effektors 3 fixiert und mit dem Übertragungsstirnrad 22 in Kämmeingriff ist. Würde demzufolge das Übertragungsstirnrad 22 bei dieser Betätigungsart, d.h. der Verschwenkbetätigung, feststehen, würde die Schwenkbewegung des Instrumentenkopfs 3 ein gleichläufiges Abrollen des Abtriebsstirnrads 21 an dem Übertragungsstirnrad 22 bewirken und somit zwangsläufig zu einer überlagerten Rotationsbewegung des Effektors 3 führen.

Wie vorstehend jedoch ausgeführt wurde, wird die Zahnradwelle 26 bei einer Schwenkbewegung des Griffstücks 1 zusammen mit dem Kulissenelement 8 mitgedreht und treibt dabei die Antriebswelle 24 innerhalb des Schubrohrs 12 an. Die Übersetzung zwischen der Zahnradwelle 26 und der Antriebswelle 24 ist dabei so berechnet, dass das Übertragungszahnrad 22 durch die Anriebswelle 24 um einen solchen Drehwinkel gedreht wird, der dem Drehwinkel entspricht, welcher durch das Abtriebszahnrad 21 bei einer entsprechenden Abwinkelung des Instrumentenkopfs 4 hervorgerufen wird, wodurch sich beide Drehungen aufgrund ihrer Gegenläufigkeit kompensieren. Bei dieser Konstellation wird die Relativposition zwischen dem Übertragungsstirnrad 22 und dem Abtriebsstirnrad 21 auch während der Abwinkelungsbewegung des Instrumentenkopfs 4 beibehalten, so dass der Effektor 3 in jeder Abwinkelungsposition des Instrumentenkopfs 4 bzw. während einer Abwinkelungsbewegung in seiner augenblicklichen Rotationsposition bezüglich des Instrumentenkopfs 4 gehalten wird.

Um eine Betätigung des Effektors 3 d.h. dessen Funktion selbst zu bewirken, ist in dem vorliegenden bevorzugten Ausführungsbeispiel der am Griffstück 1 schwenkbar gelagerte Hebel 7 vorgesehen. Wie bereits vorstehend zu den Fig. 6a-6c ausgeführt wurde, ist der Hebel 7 über ein nicht weiter dargestelltes Umlenkgetriebe oder einen entsprechenden Gelenkmechanismus mit dem in der Drehwelle 24 gelagerten Schubstab 35 wirkverbunden, welcher sich bei einer entsprechenden Betätigung des Hebels 7 relativ zur Drehwelle 24 axial hin- und herbewegt. Auch ein einfacher Bowdenzug oder ein Umlenkhebel wäre für eine Kraftübertragung auf den Schubstab 35 denkbar.

Die Fig. 6a zeigt nunmehr die Relativlage des Schubstabs 35 sowie des Schubbolzens 32 in 0°-Abwinkelposition des Instrumentenkopfs 4 bei geöffneter Zange, die Fig. 6b zeigt die Relativlage des Schubstabs 35 sowie des Schubbolzens 32 in ca. 45°-Abwinkelposition des Instrumentenkopfs 4 bei geöffneter Zange und die Fig. 6c zeigt die Relativlage des Schubstabs 35 sowie des Schubbolzens 32 in ca. 45°-Abwinkelposition des Instrumentenkopfs 4 bei geschlossener Zange.

Wie aus den Fig. 6a-6c zu entnehmen ist, wird der Schubbolzen 32 durch die Vorspannkraft der Feder 33 in ständiger Anlage mit der abgeschrägten oder gefasten distalen Stirnfläche des Schubstabs 35 gehalten. Bei einer Verschiebung des Schubstabs 35 in Richtung Instrumentenkopf 4 im Fall einer 0°-Abwinkelung des Instrumentenkopfs 4 gemäß der Fig. 6a wird der Schubbolzen 32 mit gleicher Geschwindigkeit und Wegstrecke wie der Schubstab 35, d.h. ohne Übersetzung, entgegen der Vorspannkraft der Feder 33 verschoben, wodurch die daran angelenkte Zangenbacke 30 in Schließrichtung verschwenkt wird.

An dieser Stelle sei darauf hingewiesen, dass durch die Verschiebetätigkeit des Schubstabs 35 der Schubbolzen 32, d.h. insbesondere der Mittelpunkt des Bolzenkopfradius nur annähernd auf der Schwenkachse des Instrumentenkopfs 4 verbleibt, sich also bei einer Abwinkelungsbewegung des Instrumentenkopfs 4 auf einer Art Kreisbahn bewegt. Wie jedoch eingangs der Figurenbeschreibung bereits ausgeführt wurde, sind die Stellwege für ein Öffnen und Schließen beispielsweise der Zange aufgrund der eingestellten Übersetzungen so gering, dass der Kreisbahnradius zwar theoretisch berechenbar ist, jedoch bereits schon aus fertigungstechnischen Gründen (Eigenelastizität der verwendeten Materialien, Maßtoleranzen und Spiel an den Gelenkverbindungen und Getriebeteilen) keinen relevanten Einfuß auf Zangenstellung hat. In anderen Worten ausgedrückt, wird die Zangenstellung durch die Position des Hebels 7 bestimmt, welcher wiederum von einer Bedinerperson gehalten wird und damit beispielsweise auch unkontrollierbaren Handbewegungen (Zitterbewegungen) unterliegt. Derartige aufgrund manueller Betätigungen erzeugte Störungen sind im Vergleich zu jenen Störungen, welche durch die vorstehend beschriebene Kreisbahnbewegung erzeugt werden, wesentlich größer und daher praktisch alleinig maßgeblich.

D.h. unabhängig von der aktuellen Position des Schubstabs 35 bzw. des Schubbolzens 32 bewirkt eine Abwinkelung des Instrumentenkopfs 4 generell nicht nur ein Verschwenken des Schubbolzens 32 bezüglich des Schubstabs 35 sondern auch ein geringes Abgleiten des Bolzenkopfs 34 auf der abgeschrägten Stirnfäche des Schubstabs 35. Durch diese geringe Abgleitbewegung bleibt der Anlagekontakt des Schubbolzens 32 auf der Stirnfläche erhalten, wobei jedoch nur eine solche Ausgleichs-Längsbewegung des Schubbolzens 32 in Folge seiner Abgleitbewegung erfolgt, die zu keiner praktisch relevanten Veränderung der Schließ- oder Öffnungsposition am Effektor 3 führt. Gleichzeitig jedoch wird eine Art Kraftumlenkmechanismus geschaffen, um eine Längsbewegung des Schubstabs 35 in eine Längsbewegung des nunmehr im Winkel zum Schubstab 35 stehenden Schubbolzens 32 durch die Abschrägung der Schubstab-Stirnfläche zu bewirken.

In anderen Worten ausgedrückt, wird der Schubstab 35 bei einer Abwinkelungsposition > 0° gemäß der Fig. 6b in Schließrichtung des Effektors 3 verschoben, wie dies in der Fig. 6c dargestellt ist, gleitet die abgeschrägte Stirnfläche des Schubstabs 35 längs am Bolzenkopf 34 vorbei und übt dabei eine Vorschubkraft auf den Schubbolzen 32 aus, der sich dementsprechend in Schließrichtung des Effektors 3 bewegt.

## Patentansprüche

1. Chirurgisches Instrument mit einem Griffstück (1), das an einem proximalen Endabschnitt eines Rohrschaft (2) angelenkt ist, an dessen distalem Endabschnitt ein einen Effektor (3) um dessen Längsachse drehbar lagernden Instrumentenkopf (4) neigbar angelenkt ist, wobei der Effektor (3) zumindest ein schwenkbares Eingriffselement (30) hat, welches über einen Effektor-Betätigungsgetriebezug mittels des Griffstücks (1) betätigbar ist,
wobei
der Effektor-Betätigungsgetriebezug einen im Rohrschaft (2) verschiebbar angeordneten Schubstab (35) hat, der im Anlenkbereich zwischen Instrumentenkopf (4) und Rohrschaft (2) mit einem im Instrumentenkopf (4) und/oder Effektor (3) verschiebbar gelagerten Schubbolzen (32) in Anlage ist, der mit dem Eingriffselement (30) wirkverbunden ist, wobei der Schubstab (35) sowie der Schubbolzen (32) in einer Neigungsposition des Instrumentenkopfs (4) zum Rohrschaft (2) von 0° koaxial zueinander ausgerichtet sind.

2. Chirurgisches Instrument nach Anspruch 1, **dadurch**
**gekennzeichnet, dass**
der Schubstab (35) eine distale Stirnfläche hat, welche den Eingiffsabschnitt mit dem Schubbolzen (32) bildet und welche unter einem vorbestimmten Winkel von vorzugsweise 45° abgeschrägt ist.

3. Chirurgisches Element nach Anspruch 2, **dadurch**
**gekennzeichnet, dass**
der Schubbolzen (32) einen kugelförmigen Bolzenkopf (34) hat, der mit der Stirnfäche des Schubstabs (35) in Anlage ist.

4. Chirurgisches Element nach Anspruch 3, **dadurch**
**gekennzeichnet, dass**
der Schubbolzen (32) mittels einer Feder (33) gegen die Stirnfläche des Schubstabs (35) vorgespannt ist.

5. Chirurgisches Element nach Anspruch 3 oder 4, **dadurch**
**gekennzeichnet, dass**
der Mittelpunkt des Bolzenkopfes (34) in einer Neigungsposition des Instrumentenkopfs (4) zum Rohrschaft (2) von 0° auf der Neigungsachse des Instrumentenkopfs (4) liegt.

6. Chirurgisches Element nach Anspruch 2 oder 3, **dadurch**
**gekennzeichnet, dass**
die Abschrägung der schubstabseitigen Stirnfläche in die Neigungsrichtung des Instrumentenkopfs (4) ausgerichtet ist.

## Claims

1. Surgical instrument with a handle (1) hinged to the proximal end section of a tubular shaft (2) with an instrument head (4) carrying an effector (3) rotatably about the effector's longitudinal axis tiltably hinged to the tubular shaft's distal end section, the effector (3) having at least one pivotable operative element (30) which is operable by means of the handle (1) through an effector-operating gear train, the effector-operating gear train having a push bar (35) which is displaceably arranged inside the tubular shaft (2) and which in the hinge region between instrument head (4) and tubular shaft (2) bears on a push bolt (32) which is displaceably mounted within the instrument head (4) and/or effector (3) and is actively connected to the operative element (3), the push bar (35) and push bolt (32) being coaxially aligned with each other in the 0° tilt position of the instrument head (4) with respect to the tubular shaft (2).

2. Surgical instrument according to Claim 1, **characterized in that** the push bar (35) has a distal end face which forms the zone of engagement with the push bolt (32) and which slopes at a predetermined angle of preferably 45°.

3. Surgical element [sic] according to Claim 2, **characterized in that** the push bolt (32) has a ball-shaped bolt-head (34) which bears on the end face of the push bar (35).

4. Surgical element [sic] according to Claim 3, **characterized in that** the push bolt (32) is biased against the end face of the push bar (35) by means of a spring (33).

5. Surgical element [sic] according to Claim 3 or Claim 4, **characterized in that** the centre of the bolt-head (34) lies on the tilt axis of the instrument head (4) in the 0° tilt position of the instrument head (4) with respect to the tubular shaft (2).

6. Surgical element [sic] according to Claim 2 or Claim 3, **characterized in that** the slope of the push bar end face is orientated in the tilting direction of the instrument head (4).

## Revendications

1. Instrument chirurgical, comprenant une poignée (1) qui est montée en articulation sur un tronçon terminal proximal d'une tige tubulaire (2), une tête d'instrument (4) qui sert d'appui à un effecteur (3) avec faculté de rotation autour de son axe longitudinal étant montée en articulation avec possibilité d'inclinaison sur le tronçon terminal distal de celle-ci, sachant que l'effecteur (3) a au moins un élément d'engagement pivotant (30) qui peut être actionné par l'intermédiaire d'un train d'engrenages d'actionnement d'effecteur au moyen de la poignée (1), dans lequel le train d'engrenages d'actionnement d'effecteur a une tige de poussée (35) qui est agencée dans la tige tubulaire (2) avec faculté de translation et qui, dans la zone d'articulation entre la tête d'instrument (4) et la tige tubulaire (2), est en contact avec un goujon de poussée (32) monté avec faculté de translation dans la tête d'instrument (4) et/ou dans l'effecteur (3), ledit goujon étant relié efficacement avec l'élément d'engagement (30), sachant que la tige de poussée (35) ainsi que le goujon de poussée (32) sont orientés coaxialement de 0° l'un par rapport à l'autre dans une position d'inclinaison de la tête d'instrument (4) par rapport à la tige tubulaire (2).

2. Instrument chirurgical selon la revendication 1, **caractérisé en ce que** la tige de poussée (35) a une surface frontale distale qui forme le tronçon d'engagement avec le goujon de poussée (32) et qui est biseautée sous un angle prédéterminé de préférence 45°.

3. Instrument chirurgical selon la revendication 2, **caractérisé en ce que** le goujon de poussée (32) a une tête de goujon sphérique (34) qui est en contact avec la surface frontale de la tige de poussée (35).

4. Instrument chirurgical selon la revendication 3, **caractérisé en ce que** le goujon de poussée (32) est précontraint contre la surface frontale de la tige de poussée (35) au moyen d'un ressort (33).

5. Instrument chirurgical selon l'une ou l'autre des revendications 3 et 4, **caractérisé en ce que** le centre de la tête de goujon (34) est disposé sur l'axe d'inclinaison de la tête d'instrument (4), dans une position d'inclinaison de 0° de la tête d'instrument (4) par rapport à la tige tubulaire (2).

6. Instrument chirurgical selon l'une ou l'autre des revendications 2 et 3, **caractérisé en ce que** le biseautage de la surface frontale du côté tige de poussée est orienté dans la direction d'inclinaison de la tête d'instrument (4).
